# EUROPEAN PATENT APPLICATION

(11) **EP 2 286 763 A1**
(43) Date of publication of application: **23.02.2011**
(21) Application number: 10172666.9
(22) Date of filing: 12.08.2010
(51) Int. Cl.: A61F 2/16

(54) **Cassette for an intraocular lens, injector device with such a cassette, method for storing an intraocular lens in a cassette and method for advancing an intraocular lens out of a cassette**

(30) Priority: 18.08.2009 GB 0915097
(71) Applicant: Carl Zeiss Meditec SAS, 17053 La Rochelle Cedex 9 (FR)
(72) Inventor: Pankin, Dmitry, 81737, München (DE); Raquin, Vincent, 17000, La Rochelle (FR); Rathert, Brian, 17000, La Rochelle (FR)
(74) Representative: Lechner, Armin Anton

(57) **Abstract**

The invention relates to a cassette for containing an intraocular lens (11), wherein the cassette (8) is formed for inserting in a lens injector device (1), and has an entry region (12) for a plunger (4) of the injector device (1) and an exit region (15) for the lens (11), **characterized in that** the cassette (8) includes a movement channel (121) for the plunger (4), wherein a longitudinal axis (A) of the movement channel (121) and a center-plane (I) of the intraocular lens (11) are oriented inclined to each other in the state loaded in the cassette (8) in a rest-position of the intraocular lens (11). The invention also relates to a lens injector device (1) including a cassette (8). Furthermore, the invention relates to a method for storing an intraocular lens (11) in a cassette (8) as well as a method for advancing an intraocular lens (11) out of a cassette (8).

## Description

### Technical field

The invention relates to a cassette for containing an intraocular lens, wherein the cassette is formed for inserting into an injector device, wherein the injector device is formed for introducing the intraocular lens into the eye. The cassette has an entry region for the plunger of the injector device and an exit region for the lens. Moreover, the invention relates to an injector device with such a cassette. Furthermore, the invention relates to a method for storing an intraocular lens in a cassette as well as a method for advancing an intraocular lens out of a cassette.

### Prior art

An intraocular lens is an eye implant, which is inserted into the eye if the original natural lens has to be replaced due to the produced defective vision. In such cataract surgeries, through a small incision in the eye, the natural lens is fragmented and sucked off. Through such small incisions in the eye, then, the intraocular lens is inserted. Such an intraocular lens is known in manifold configuration and usually has an optic part and plural haptic parts. The intraocular lens is positionally stably held in the eye by the haptic parts.

For introducing such intraocular lenses into the eye, an injector device is used, wherein, here too, various configurations are known. From US 2005/0125000 A1, such an injector device is known. It has a plunger, which can be movably displaced in a tube. A cassette receiving the intraocular lens can be inserted into this tube. The plunger engages the cassette through an entry region and engages the optic part of the lens, which is disposed in horizontal direction in the cassette. Then, by means of the plunger, the lens is advanced out of the cassette through an exit region and advanced into a tapering injection channel following the tube of the injector device. There, the lens is automatically rolled up.

### Presentation of the invention

It is the object of the present invention to provide a cassette and an injector device as well as a method for storing an intraocular lens in a cassette and a method for advancing an intraocular lens out of a cassette, by which or in which damage of the intraocular lens, in particular of the haptic part, can be prevented. In particular, undesired mechanic stresses and force effects on the lens also are to be avoided.

This object is solved by a cassette having the features according to claim 1, an injector device having the features according to claim 15, a method having the features according to claim 17 and furthermore by a method having the features according to claim 20.

A cassette according to the invention is formed for receiving an intraocular lens, wherein the cassette is formed for inserting into a lens injector device and has an entry region for a plunger of the injector device and an exit region for the lens. The cassette includes a movement channel for the plunger, wherein a longitudinal axis of the movement channel and a center-plane of the intraocular lens are oriented inclined to each other in the state loaded into the cassette in a rest-position of the intraocular lens. This implies that the horizontal plane and the longitudinal axis are disposed in an angle greater than 0° to each other, in particular disposed in an angle between 5° and 50°. By the inclined arrangement, undesired mechanic stresses of the lens can be prevented and undesired force effects of the plunger on specific regions of a haptic part of the lens upon advancement out of the cassette can be avoided.

Preferably, the longitudinal axis extends in a horizontal plane of the cassette, and the intraocular lens is positioned inclined with respect to the horizontal plane in a rest-position in the cassette. It can also be provided that the center-plane of the lens is parallel to or the same as the horizontal plane of the cassette and the longitudinal axis of the movement channel is disposed in an angle greater than 0° thereto.

Preferably, a position holding device for the intraocular lens is formed, by which an inclined rest-position of the intraocular lens in the cassette can be held.

In particular, the cassette includes a cover part and a base part movable relatively thereto, and the position holding device is formed by elements of the cover part and/or the base part, wherein, for this, the cover part and/or the base part have a shaping at least in certain regions on their surfaces facing the lens, which result in an inclined rest-position of the lens in the closed state of the cassette with the lens received.

In particular, the position holding device is formed such that a tolerance of movement is formed for the intraocular lens in the rest-position. This implies that presetting for the lens is provided by the position holding device, in which the lens can minimally move. Therefore, the position holding device is not an exactly fitting configuration and not form-closing the on lens. Rather, the lens is virtually in a free state within the position holding device. However, the tolerances of movement are preferably 0.1 mm at the maximum at least at one location. This implies that at least at one location virtually a shaped shell is formed by the position holding device, which restricts the mobility of the lens to the top or the bottom to a maximum of 0.1 mm. Especially when a liquid is additionally loaded into the cassette, the lens can virtually float, however, it is held in the inclined position within the tolerances of movement by the position holding device. Excessive friction or excessive mechanic effects on the lens in the rest-position can thereby be avoided.

Preferably, the position holding device is formed such that the lens is disposed inclined to the bottom in the rest-position based on the end of the lens facing the entry region of the cassette. Thereby, the accessibility of the plunger to the lens can be achieved in particularly advantageous manner, and the advancement can thereby be achieved in simplest manner and with the lowest mechanical influences on the lens.

Preferably, the rest-position of the intraocular lens in the cassette by the position holding device is formed such that the center-plane and the longitudinal axis, in particular a horizontal plane having the longitudinal axis, intersect in the region of an outer edge of an optic part of the intraocular lens, which has a recess in a haptic part, or a first section of a haptic part of the intraocular lens directly abutting on an edge of the optic part. Thereby, the functionality and the smooth-running cooperation of the components for contacting and advancing the lens are particularly effective, precise and low-wear.

The haptic parts of the lens can be disposed angled with respect to the optic part. It can also be provided that the haptic parts and the optic parts are preferably disposed in one plane.

Preferably, the position holding device has at least one form element, which has a lay-on surface, on which the intraocular lens can be laid with a haptic part in the rest-position. Thus, this serves for the lens to lay on there due to the possible clearance of motion of the lens in the position holding device and optionally depending on the amount of liquid in the cassette. However, it can also be provided that it does not rest there, in particular if correspondingly much liquid is present.

Preferably, the cassette, in particular the position holding device, is formed for receiving an intraocular lens, which has two haptic parts, which are formed on opposing positions of an optic part, wherein the rest-position of the lens in the cassette is formed such that the longitudinal axis of the movement channel extends through the two haptic parts or is an axis of symmetry of the lens through the haptic parts in one plane with the longitudinal axis in case of a position of the lens inclined with respect to the horizontal plane. Therefore, the specific lens is also disposed in a specific position with regard to the orientation of the haptic parts in the cassette, whereby a particularly advantageous mechanical principle of action for advancing the lens can be achieved in conjunction with the inclined position, in which undesired mechanical force effects on specific regions of the haptic parts can be avoided.

By this inclined arrangement, a position can be provided, by means of which the intraocular lens can be taken from the cassette in more gentle manner by means of the plunger, and moreover can also be displaced with lower mechanic influences and without undesired mechanic stress conditions.

In particular, thereby, it can be achieved that haptic parts of the intraocular lens are not exposed to undesired force effects by the plunger if the intraocular lens can be inserted into the cassette only in the manner that at least one haptic part is at least partially disposed in the movement path of the plunger.

Just in configurations of a cassette, in which the intraocular lens is loaded into the cassette in plane manner and a haptic part of the intraocular lens faces or is turned towards the entry region of the cassette, through which the plunger is introduced into the cassette, and thus compulsorily, the plunger exclusively can only engage the soft outer haptic part for advancing out the lens during its horizontal movement, this is disadvantageous with regard to the damage of this haptic part.

With the cassette according to the invention, this can be avoided since engagement with undesired locations of the haptic part of the intraocular lens by the plunger can be avoided due to the non planar arrangement, but the arrangement of the intraocular lens in the cassette inclined with respect to the horizontal plane. Thereby, undesired deformations or damages of the intraocular lens, particularly of the exterior locations of the haptic part, can be avoided.

By this inclination of the intraocular lens in the rest-position in the cassette, engaging positions for the plunger for advancing the intraocular lens out of the cassette can be allowed, which are formed substantially more unsusceptible and robust with regard to the mechanic influence and mechanic stress conditions by the plunger.

Thereby, the damage of the intraocular lens, in particular of haptic parts of the intraocular lens, at least can be considerably reduced.

Preferably, the form element of the cassette is disposed on a base part of the cassette. This allows a particularly suitable positioning of the intraocular lens with regard to the inclined arrangement in the cassette.

Preferably, the moving direction of a plunger of the injector device for advancing the intraocular lens out of the cassette extends in the horizontal plane or parallel to the horizontal plane. Thus, in particular, the intraocular lens is also disposed inclined with respect to the moving direction of the plunger. This is a particularly advantageous configuration with regard to the mechanic contacts of the plunger with the intraocular lens in order to be able to avoid undesired damages to the intraocular lens, in particular of haptic parts.

Preferably, in the rest-position of the intraocular lens and of the cassette, a center-plane of the intraocular lens is disposed in an angle with respect to the horizontal plane such that the lens is disposed inclined downwards based on the end of the lens facing the entry region of the cassette. By this angle > 0°, these defined planes are disposed intersecting each other. Just this center-plane and the inclined arrangement thereof to the horizontal plane present a particularly advantageous positioning with regard to the suitable mechanic points of contact for the engaging plunger. Exactly if the intraocular lens would be positioned with a haptic part in the moving direction of the plunger, by this inclination, it can be achieved that a soft section of the haptic part is no longer the main point of engagement of the plunger for advancing the intraocular lens out of the cassette.

Preferably, the intraocular lens is formed such that the center-plane extends through the optic part and through all of the haptic parts. Preferably, thus, the optic part of the intraocular lens and the haptic parts are disposed and formed in one plane.

It proves particularly preferred if the rest-position of the intraocular lens is formed such that the center-plane of the intraocular lens and the horizontal plane intersect at a place formed in a region on the circumferential edge of the optic part of the intraocular lens. On this outer edge of the optic part, a first section of the haptic part can be disposed, which is considerably more stable with regard to the engagement and abutting possibilities of a plunger than a second section of this haptic part disposed spaced from the optic region. Exactly this positioning with regard to these specific regions of intersection between the center-plane and the horizontal plane allows a particularly advantageous configuration with regard to the particularly gentle contact of the plunger with the intraocular lens and the particularly gentle advancement of the intraocular lens out of the cassette.

Preferably, the form element has an opening for guiding-through the plunger of the injector device.

Therefore, through the entry region of the cassette, the plunger is also advanced through by the form element upon advancing the intraocular lens out of the cassette, whereby particularly advantageous points of engagement of the plunger with the intraocular lens and a particularly functional advancing-out can be achieved with regard to the inclined positioning. Moreover, the cassette can be formed in compact and installation space-minimized as well as mechanically stable manner.

Preferably, in the rest-position in the cassette, by the form element, the intraocular lens is higher disposed at the end of the cassette facing the entry region than at the end turned away from the entry region or the end facing the exit region, respectively. Therefore, considered from the entry region in the direction of the advancing-out direction and thus considered in the direction of the exit region, the inclination is preferably tilted to the bottom. This is advantageous with regard to the contact of the plunger with the intraocular lens and in particular advantageous with regard to undesired movements of the lens in the cassette upon the further procedure of advancing-out.

Preferably, the form element has a lay-on surface, on which the intraocular lens rests with a haptic part in the rest-position. By this configuration, the rest-position can securely be held and undesired loads on the intraocular lens can be avoided. By this lay-on surface, moreover, resting with an area as large as possible can be provided, whereby, here too, damages thereof can be avoided.

Preferably, a haptic part of the intraocular lens has a recess or a hole, by which a first section of the haptic part abutting the optic part of the lens, in particular a first section abutting a circumferential edge of the optic part, is formed. Moreover, by the recess, a second section of the haptic part spaced from the optic part is formed, which is relatively soft an deformable with regard to its configuration and which is therefore not contacted by the plunger on the peripheral side due to the specific rest-position in order to advance out the intraocular lens. Exactly this contact of the plunger with the outer edge of this second section of the haptic part is avoided by the present invention with respect to the cassette such that undesired deformations or undesired mechanic stresses and loads of this section of the haptic part can be avoided. In particular, the second section of the haptic part rests on the lay-on surface when the intraocular lens is disposed in its rest-position in the cassette.

By this configuration of the intraocular lens with these specific configurations of the haptic part and the inclined arrangement in the cassette, upon advancing-out and contacting the plunger, it can be achieved that this second section of the haptic part virtually remains nearly unaffected by the plunger and is in no case wiped on the peripheral side, but at most on the bottom or on the top, and the plunger slides along this bottom or top. An undesired mechanic deformation by contact with the plunger on the peripheral side at this second section of the haptic part can thus be avoided. By this position and configuration of the intraocular lens in the cassette, the haptic part can virtually be swept on the bottom or top with the plunger in particular with respect to its second section, and thus is no longer exposed to undesired mechanic loads in this respect.

Preferably, the intraocular lens is disposed in the cassette in its rest-position such that the second section of the haptic part is disposed above the movement path of the plunger. Thereby, the outer edge of the second section of the haptic part cannot be contacted by the plunger and, at best, contacting and sliding along the bottom of the second section of the haptic part can be effected by the plunger. This advantageously contributes to the avoidance of undesired deformations and mechanic loads upon advancing the intraocular lens out of the cassette.

For advancing the lens out of the cassette the plunger preferably engages at least partially in the recess of the haptic part.

Preferably, the rest-position is formed such that for advancing the lens out of the cassette, the plunger is disposed in non-contacting manner with the outer edge of the second section of the haptic part.

Preferably, in a first phase of advancing-out, the plunger is contacted with a bottom of the second section of the haptic part for lifting the lens. Thus, the plunger relatively gently slides along this bottom and lifts the intraocular lens upwards from its resting position on the lay-on surface of the form element. By the plunger only engaging the bottom of this second section and sliding along it, undesired deformation of a second section of the haptic part is not caused. In a second phase of advancing-out, the plunger is preferably contacted with the first section of the haptic part and/or the optic part. After lifting, thus, furthermore, a position of contact of the plunger with the intraocular lens is reached, which is considerably more robust than the second section of the haptic part with respect to mechanic loads. Thereby, further advancing-out in this region of contact of the plunger with the intraocular lens can be ensured.

Preferably, the lens is disposed in its rest-position in the cassette such that the second section of the haptic part is disposed above the movement path of the plunger, in particular a bottom of the second section rests on the plunger after releasing the rest-position of the lens.

Preferably, the lay-on surface of the form element is respectively limited by an elevation at the front and at the back. In this respect, webs can be provided, between which the lay-on surface extends. By these elevations, the rest-position and the laid-on intraocular lens, in particular the second section of the haptic part of the intraocular lens, can be retained in particularly secure manner.

Preferably, the optic part of the intraocular lens is disposed in non-contacting manner with the form element in the rest-position in the cassette. It proves particularly advantageous if the optic part of the lens is positioned substantially completely freely floating in the cassette in the rest-position. It can be provided that the optic part of the lens rests on a base part of the cassette with maximum 10 % of its area in the rest-position in the cassette and else is disposed in non-contacting and freely floating manner with the cassette. In this respect, thus, only contacting resting states of a first haptic part on the form element on the one hand and of a further haptic part on a base part are provided, wherein at this place of the base part, the optic part formed adjacent thereto can still slightly rest on the base part also in direct manner.

Such a freely floating large-area arrangement of the intraocular lens allows a particularly target-oriented and gentle contact by means of the plunger and a particularly smooth-running lifting of the lens from its rest-position. Thereby, particularly gentle advancing-out formed with mechanic loads as low as possible can be effected.

Preferably, the angle of inclination between the horizontal plane and the lens, in particular the center-plane of the lens, is formed between 5° and 40°, in particular 5° and 10°, especially between 7° and 8°. They are particularly advantageous angular ranges with regard to a mechanically stable position formed with loads as low as possible on the one hand, and a particularly advantageous arrangement with regard to the simple and low-effort as well as low-load advancement of the lens out of the cassette on the other hand.

Preferably, the intraocular lens has two haptic parts, which are disposed on the edge on opposing sides of the optic part.

Furthermore, the invention relates to a lens injector device, which has a cassette according to the invention or an advantageous development thereof. The lens injector device includes a mobile movable plunger, which is formed to advance the intraocular lens out of the cassette into an injection channel of the lens injector device.

Preferably, the rest-position of the lens in the cassette is formed by the position holding device such that a front lateral edge of a haptic part of the lens facing the movement channel of the plunger is disposed free from bending outside of the movement path of the plunger. Therein, the rest-position particularly includes the phases, in which the lens is disposed in the cassette before contacting the plunger, and in particular the phase, in which the plunger approaches the lens and then contacts it. Thus, even just before contacting the lens by the plunger, bending of the lens by the position holding device or another part of the cassette is not performed, in order to optionally be able to allow the accessibility of the plunger to a certain location, in particular the optic part and/or a specific region of the haptic part of the lens. In conjunction with the inclination between the lens and the longitudinal axis of the movement channel and thus also the piston, thus, a highly matched guide of movement can be achieved.

Preferably, the injection channel is formed as a hollow truncated cone, which thus tapers such that upon advancing the intraocular lens into this injection channel, automatic folding and thus rolling-up of the intraocular lens can be generated.

Furthermore, the invention relates to a method for storing an intraocular lens in a cassette, wherein the cassette is formed for inserting into an injector device and the injector device is formed for introducing the intraocular lens into the eye. The cassette is formed with an entry region for a plunger of the injector device and an exit region for the intraocular lens. The cassette includes a movement channel for the plunger, wherein a longitudinal axis of the movement channel and a center-plane of the intraocular lens are oriented inclined to each other in the state loaded in the cassette in a rest-position of the intraocular lens.

Preferably, the longitudinal axis extends in a horizontal plane of the cassette and the intraocular lens is positioned inclined with respect to the horizontal plane in a rest-position in the cassette, in particular a center-plane of the intraocular lens is disposed in an angle with respect to the horizontal plane such that the lens is disposed inclined to the bottom based on the end of the lens facing the entry region of the cassette.

Preferably, the intraocular lens has two haptic parts formed on opposing positions of an optic part, wherein the rest-position of the lens in the cassette is formed such that the longitudinal axis of the movement channel extends through the two haptic parts or an axis of symmetry of the lens extends through the haptic parts in a plane with the longitudinal axis in case of a position of the lens inclined with respect to the horizontal plane. Preferably, in the rest-position of the intraocular lens in the cassette, a center-plane of the intraocular lens is disposed in an angle with respect to the horizontal plane such that the lens is disposed inclined to the bottom based on the end of the lens facing the entry region of the cassette.

In particular, in the rest-position, the intraocular lens can be laid on a lay-on surface of the form element with a haptic part, in particular laid on this lay-on surface with a second section of the haptic part disposed in non-contacting manner to the optic part of the intraocular lens. Again, this depends on the liquid in the cassette, in particular on the liquid wetting the lens.

Preferably, in the haptic part of the intraocular lens, a recess is formed, by which a first section of the haptic part adjoining to the optic part of the lens is formed, and a second section of the haptic part spaced from the optic part is formed, wherein the lens is positioned in the cassette such that the second section can be laid on the lay-on surface in the rest-position of the lens in the cassette.

Preferably, the lay-on surface is respectively limited at the front and at the back by an elevation of the form element, and in the rest-position of the lens in the cassette, the haptic part of the lens can be laid on the lay-on surface between the elevations, wherein in particular the second section of the haptic part is laid on this lay-on surface between the elevations.

Preferably, the optic part of the lens is disposed in non-contacting manner with the form element in the rest-position in the cassette. In particular, the optic part of the lens is disposed substantially or completely freely floating in the rest-position.

Furthermore, the invention relates to a method for advancing an intraocular lens out of a cassette, wherein the cassette is formed according to the invention or according to an advantageous embodiment of the invention. In the method, a plunger of a lens injector device is advanced to the lens through an entry region of the cassette, and upon advancing the lens out of the cassette through the exit region, the plunger is moved in non-contacting manner with an edge or border of a second section of the haptic part of the lens. Therefore, it can be ensured that high mechanic stresses and loads on the edge of the second section of the haptic part will not occur by the plunger, and thus undesired deformations and damages to the haptic part can also be avoided in this respect.

The edge or border of the second section extends between an upper side or an upper surface and a bottom side or a bottom surface of said second section of a haptic part.

It can be provided that upon advancing the lens out of the cassette, only the first section of the haptic part of the lens is contacted by the plunger. In such a configuration, thus, the conduction of movement of the plunger is accomplished in completely non-contacting manner with the second section of the haptic part and also in completely non-contacting manner with the optic part of the lens. The first section of the haptic part immediately disposed on the circumferential edge on the optic part is mechanically stably positioned there and serves as a suitable point of abutment for the plunger in order to be able to absorb the mechanic force effects without damage of the lens upon advancing-out.

It can also be provided that in a first phase of advancing-out, the plunger is contacted with the bottom of the second section of the haptic part and thereby the lens is lifted from its rest-position. In a second phase of advancing-out following the first phase of advancing-out, the plunger is contacted with the first section of the haptic part and afterwards the lens is advanced out. In this second phase of advancing-out, it can also be provided that the plunger is contacted with the optic part of the lens or in addition to that with the first section of the haptic part, and advancing the lens out of the cassette is performed.

After lifting from the rest-position via an elevation of the form element, preferably, the intraocular lens is advanced forwards over this elevation with the plunger and then again at least partially lowered and afterwards advanced out.

After lifting from the rest-position via an elevation of the form element, preferably, the lens is advanced forwards over this elevation with the plunger and again lowered and laid on the plunger with the bottom of the second section of the haptic part and further advanced out.

Advantageous implementations of the cassette according to the invention are to be considered as advantageous implementations of the lens injector device according to the invention. Furthermore, advantageous implementations of the cassette according to the invention are to be considered as advantageous implementations of the method according to the invention.

Further features of the invention appear from the claims, the figures and the description of figures. The features and feature combinations mentioned above in the description as well as the features and feature combinations mentioned in the description of figures alone as well as the features and feature combinations only shown in the figures are usable not only in the respectively indicated combination, but also in other combinations or alone without departing from the scope of the invention. Explained embodiments can also be combined in individual features to new embodiments.

### Brief description of the drawings

Embodiments of the invention are explained in more detail below based on schematic drawings. There show:
- Fig. 1: a perspective representation of an embodiment of a lens injector device according to the inven- tion;
- Fig. 2: an enlarged representation of a partial section of the lens injector device according to fig. 1;
- Fig. 3a: a perspective sectional representation of an embodiment of a cassette according to the in- vention with an intraocular lens disposed in the rest-position;
- Fig. 3b: a part of the representation according to fig. 3a.
- Fig. 4: a further sectional representation through the cassette according to fig. 3.
- Fig. 5: a perspective sectional representation of an- other embodiment of a cassette according to the invention with an intraocular lens disposed in the rest-position; and
- Fig. 6: a schematic representation of another embodi- ment of a cassette.

### Preferred embodiments of the invention

In the figures, similar or functionally similar elements are provided with the same reference characters.

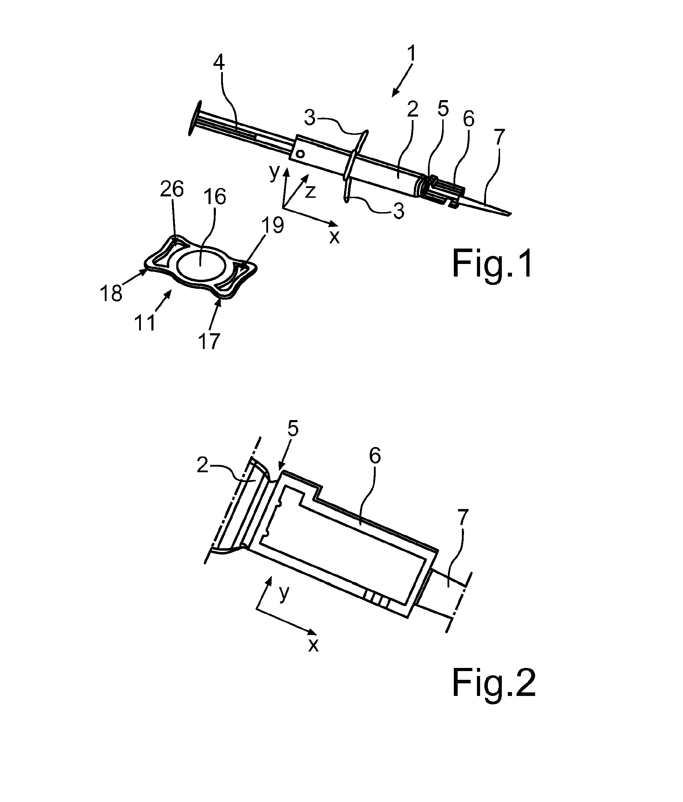

In fig. 1 and fig. 2, in a perspective representation, a lens injector device 1 is shown, which is formed for introducing an intraocular lens 11 into an eye. The injector device 1 includes a tubular hollow body 2, on which two gripping members 3 are disposed at the outside. Moreover, the injector device 1 includes a plunger 4, which is displaceable in the tubular hollow body 2 in the direction of the x-axis. On a front end 5 of the tubular hollow body 2, an receiving region 6 is formed, which is formed as a receiving frame. Following this receiving region 6, which is formed for receiving a cassette with an intraocular lens, a tapering hollow-shaped injection canula or insertion part 7 is disposed. The tubular hollow body 2 has an opening at the end 5, through which the plunger 4 can slide to advance out the lens in the cassette, which is then disposed in the receiving region 6, and to advance it into the insertion part 7. Due to the tapering configuration of the insertion part 7, the intraocular lens is automatically rolled up upon advancing into this introduction part 7.

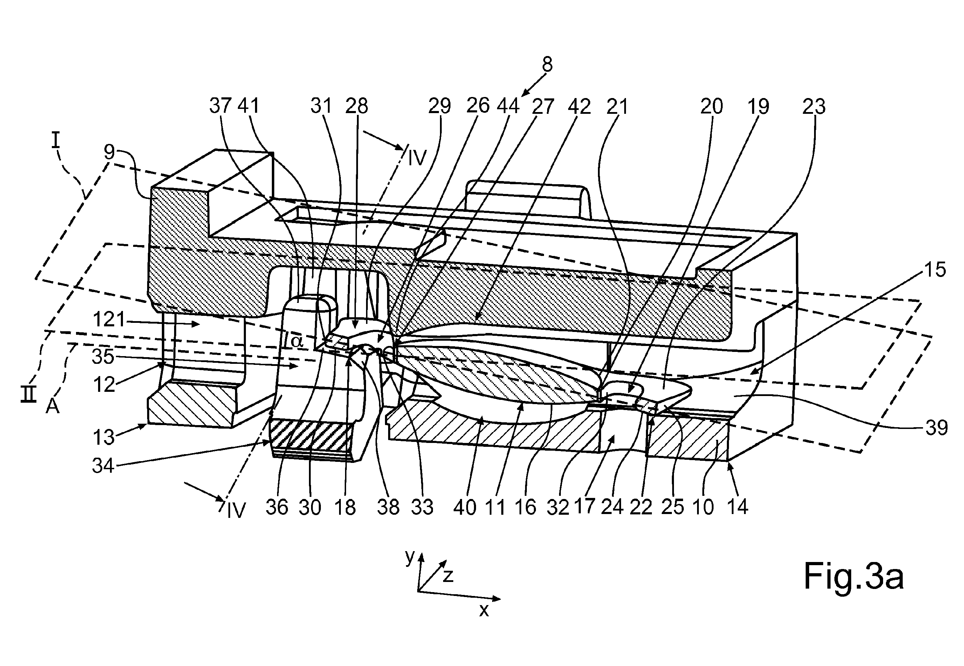

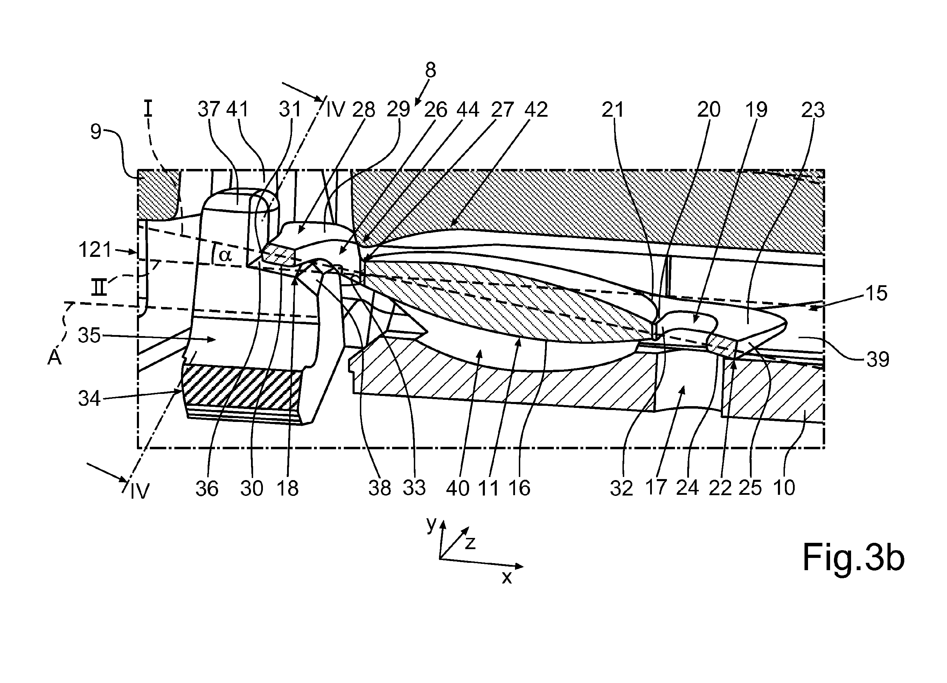

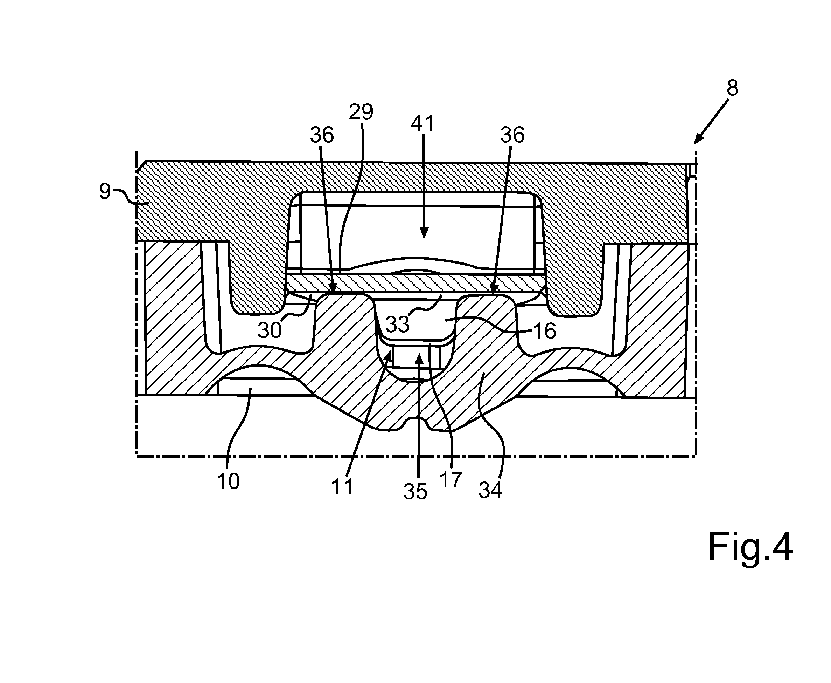

A cassette 8, as is furthermore illustrated and explained in fig. 3a and 3b and 4, can be inserted into the receiving region 6.

The cassette 8 includes a cover part 9 and a base part 10. The cover part 9 and the base part 10 are movable relatively to each other and are correspondingly openable and closeable for inserting an intraocular lens 11 into the cassette 8.

In fig. 3a, a sectional representation through the cassette 8 is shown, wherein the sectional plane extends in the x-y-plane. The cassette 8 is shown in the closed state in fig. 3a and the intraocular lens 11 is disposed in its rest-position.

The cassette 8 has an entry region 12, which follows the end 5 of the tubular hollow body 2. The plunger 4 can be advanced into the interior of the cassette 8 through the entry region 12. The entry region 12 is formed at the end 13 of the cassette 8 facing the tubular hollow body 2. At the opposing end 14 of the cassette 8 facing the insertion part 7, an exit region 15 is formed. The intraocular lens 11 can be advanced out of the cassette 8 by means of the plunger 4 through this exit region 15 and be advanced into the insertion part 7 of the injector device 1.

Thus, the plunger 4 is moved in the x-direction to advance the intraocular lens 11 out of the cassette 8. Subsequent to the entry region 12, the cassette 8 has a movement channel 121, in which the plunger 4 is guided to access the lens 11. The movement channel 121 has a longitudinal axis A, which extends horizontally and thus extends in x-direction. Correspondingly, the plunger 4 is guided horizontally. The longitudinal axis A extending in the horizontal plane II and a center-plane I of the lens 11 are disposed inclined to each other, wherein an angle α is formed.

In its shown rest-position in the cassette 8, the intraocular lens 11 is positioned inclined to the bottom. The intraocular lens 11 has a central optic part 16, on which in the embodiment a haptic part 17 and 18 are respectively disposed at opposing ends. The intraocular lens 11 only has these two haptic parts 17 and 18, wherein they are disposed at opposing ends of the optic part 16 considered in x-direction according to the representation. In the embodiment, the haptic parts 17 and 18 are not disposed angled with respect to the optic part 16, but formed in one plane with the optic part 16.

A recess 19 is formed in the first haptic part 17. A first section 20 of the haptic part 17 is formed by this recess 19. This first section 20 is relatively thin and immediately abuts the circumferential edge 21 of the optic part 16. Moreover, a second section 22 of the haptic part 17 is formed by the recess 19. It is formed spaced from the optic part 16. The first haptic part 17 has a top 23 and a bottom 24. Moreover, a lateral edge 25 is formed, which faces the exit region 15 in the rest-position.

In corresponding manner, the further haptic part 18 is formed. There too, a recess 26, which is also continuous, is formed. A first section 27 of the haptic part 18 is formed by this recess 26, which again is relatively thin and immediately abuts the circumferential edge 21 of the optic part 16. A second section 28 of the haptic part 18 spaced from the optic part 16 is formed by the recess 26. Here too, the further haptic part 18 has a top 29 and a bottom 30. Moreover, a lateral edge 31 connecting the top 29 and the bottom 30 is formed.

Moreover, a lateral edge 32 at the first section 20 and a lateral edge 33 at the first section 27 are formed by the recesses 19 and 26.

The inclined position of the intraocular lens 11 in its rest-position is **characterized in that** the end of the intraocular lens facing the entry region 12 is disposed higher in y-direction than the end of the intraocular lens 11 facing the exit region 15.

In particular, the rest-position of the intraocular lens 11 is formed such that a center-plane I of the intraocular lens 11 is disposed in an angle α with respect to a horizontal plane II. The horizontal plane II extends in the x-z-plane, wherein the moving direction of the plunger 4 is effected in the horizontal plane II or parallel thereto. The center-plane I of the intraocular lens II extends through the two haptic parts 17 and 18 as well as through the optic part 16 as it is illustrated in fig. 3a and 3b. The angle α preferably is between 5° and 40°, in particular between 10° and 20°.

In the shown implementation, the base part 10 includes a form element 34, which has a guide-through opening 35 for the plunger 4. Moreover, a lay-on surface 36 is provided, on which the end of the intraocular lens 11 facing the entry region 12 rests. In particular, the haptic part 18 and only the second section 28 thereof rests on this lay-on surface 36 with its bottom 30.

Considered in x-direction, the lay-on surface 36 is limited by a first elevation 37 and a second elevation 38, which extend upwards in y-direction. The second elevation 38 is lower than the first elevation 37. In the shown rest-position of the intraocular lens 11, thus, it is also retained by the form element 34 with respect to undesired displacements in the x-direction.

Moreover, the first haptic part 17 rests on a bottom surface 39 of the base part 10 with its bottom 24. Moreover, the base part 10 includes a well region 40, which is formed approximately on the level of the optic part 16 considered in x-direction in the rest-position of the intraocular lens 11. In the rest-position, moreover, the intraocular lens 11 is positioned such that the optic part 16 is disposed in non-contacting manner with the base part 10 and the cover part 9. At best, it can be provided that the optic part 16 rests on the bottom surface 39 with a very small surface. Thus, the optic part 16 is virtually disposed freely floating in the rest-position within the cassette 8. Preferably, the rest-position of the intraocular lens 11 is provided such that the center-plane I and the horizontal plane II intersect in the region of the optic part 16 and the first section 27 of the haptic part 18. This line of intersection between the two planes I and II is therefore locally localized to a very small region, which extends in the region of the lateral edge 33 of the first section 27 and due to the low thickness of the first section 27 in x-direction therefore also in the region of the lateral edge 21 of the optic part 16.

In particular, the lens 11 is held in the inclined rest-position by a position holding device 42. The position holding device 42 is formed by elements of the cover part 9 and the base part 10. In particular, the shapings of the cover part 9 and of the base part 10 are formed in the region of the lens 11 such that the surfaces facing the lens are adapted to the shape of the lens 11 in certain regions. However, it is essential that this shaping is formed with a tolerance of movement for the lens 11. This implies that the lens 11 is not contacted by the cover part and the base part in positive manner, but that a shaped shell is virtually formed, which allows a small movement of the lens 11 in all three spatial directions. Since, in particular, a bit of liquid also wets the lens 11, the lens is "floating" in a free state in the cassette 8, and yet is held in the inclined position. The tolerance of movement is smaller than or equal to 0.1 mm at least at one location, such that the free mobility of the lens 11 is greatly kept within limits since the inclined position is only minimally variable. In the embodiment, the location with the minimum tolerance of movement is in fig. 3a and 3b in the region of the recess 26, between an elevation 38 on the form element 34 and a convex element 44 on the cover part 9.

This positioning is particularly advantageous with regard to the contact of the plunger 4 with the intraocular lens 11 and the further procedure of advancement out of the cassette 8.

The cassette 8 has a receiving space 41 in the cover part 9, into which the lens 11, in particular the second haptic part 18, can be lifted when the lens 11 is advanced out.

In the rest-position, the second haptic part 18 is disposed above the movement path of the plunger 4. Considered in x-direction, the two haptic parts 17 and 18 are disposed one behind the other.

In fig. 4, a further sectional representation along the sectional line IV-IV in fig. 3a and 3b is shown. In this view in x-direction, the cassette 8 is virtually shown from the plunger 4. The inclined arrangement of the intraocular lens 11 formed towards the exit region 15 is recognizable.

Moreover, by the rest-position of the intraocular lens 11, it is also achieved that the plunger 4, upon contacting the intraocular lens 11, either not contacts the second section 28 of the haptic part 18 or at best only contacts the bottom 30 and wipes along it. The front lateral edge 31 of the second section 28 of the haptic part 18 is no longer contacted by the plunger 4 and therefore, a mechanic force in x-direction is no longer exerted in this respect. By this configuration, undesired force effect by the plunger 4 on this second section 28 of the haptic part 18 can be avoided. The lateral edge 31 is disposed free from bending in the rest-position of the lens 11 in the cassette 8 and particularly also during the entire approaching distance of the piston 4 until reaching the advancement contact position on the lens 11. This implies that bending of the haptic part 18 facing the plunger 4, in particular of the second section 28, by an element of the cassette 8, which also is not associated with the plunger 4, is not performed in order to be able to avoid a contact of the plunger 4 with the lateral edge 31. But bending of the haptic part 18 because of the weigth of the lens 11 is possible and is not exluded as it is with the active bending by an element as said above.

Thereby, undesired deformations upon advancing the intraocular lens 11 out of the cassette 8 can be avoided. Exactly the haptic parts, in particular the exterior second sections 28 and 22 thereof, which are soft and sensitive with regard to mechanic loads, can therefore be relieved from undesired mechanic stresses and loads.

According to the representations in fig. 3a, 3b and fig. 4, furthermore, the advancement of the intraocular lens 11 out of the cassette 8 with the plunger 4 is explained. If the cassette 8 is inserted in the receiving region 6, the plunger 4 can be introduced into the cassette 8 through the entry region 12 of the cassette 8. Therein, it then gets to the intraocular lens 11 through the movement channel 121, the guide-through opening 35 in the form element 34. Due to the inclined position and the raised positioning by means of the form element 34, the plunger 4 virtually engages below the intraocular lens 11. The position of the intraocular lens 11 can be formed by the position holding device 42 such that the plunger 4 slides in non-contacting manner past the second section 28 of the haptic part 18 and only directly engages the first section 27, in particular the edge 33, of the haptic part 18 and/or the optic part 16 of the intraocular lens 11. By the further movement of the plunger 4 in x-direction, then, the intraocular lens 11 is moved from its rest-position and advanced in x-direction to the exit region 15. Therein, the second section 28 of the haptic part 18 is released from the lay-on surface 16 and then rests on the plunger 4.

It can also be provided that the position of the intraocular lens 11 in the cassette 8 is formed by the position holding device 42 such that upon advancement into the cassette 8, the plunger 4 will also again not engage the lateral edge 31, but only contacts the bottom 30 of the second section 28 and slides along it in x-direction. Thereby, the intraocular lens 11 is first lifted in y-direction. Preferably, the lifting is such that the second section 28 is on the level of or higher than the extension of the elevation 33 in y-direction. In the further advancement of the plunger 4 in x-direction, then, the front side of the plunger 4 is directly contacted with the first section 27, for example with the edge 33, and/or directly contacted with the optic part 16 and advanced in the direction of the exit region 15.

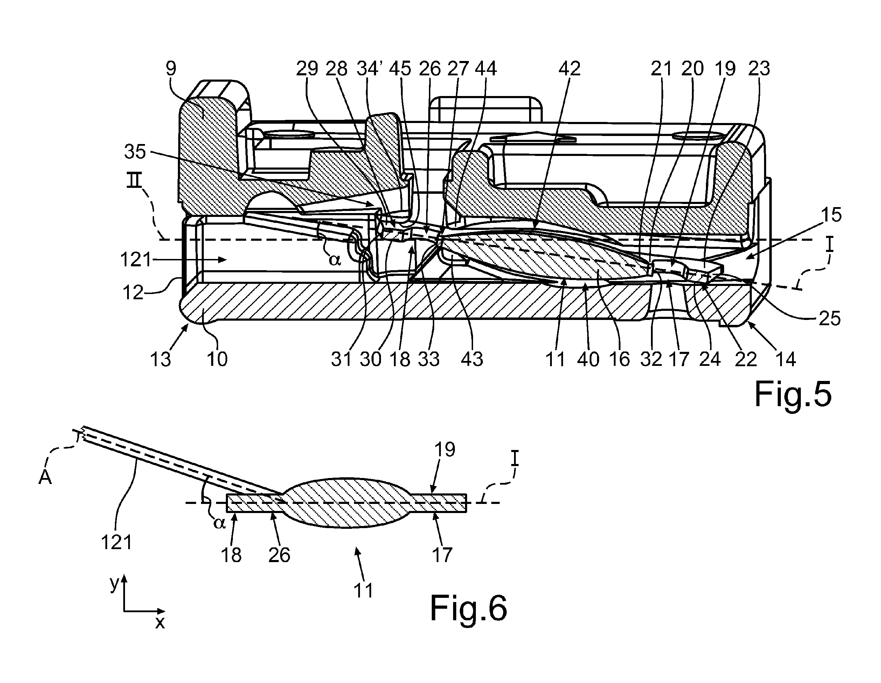

At this point, it is to be emphasized that the implementations in fig. 3a, 3b and fig. 4 are exemplary. An inverse configuration can also be provided, in which the form element 34 is disposed on the cover part 9, as it is shown in fig. 5 according to the sectional representation there. The location with the minimum tolerance of movement for the lens 11 in the position holding device 42 is formed in the region between the elements 43 on the base part 10 and the overlying cover part region. Here too, depending on the amount of liquid around the lens 11, either a lay-on surface of a raised element 43 of the base part 10 or an opposing element 44 formed at the location on the cover part 9, and/or a contact surface 45 of a form element 34' can serve as a stop for the lens 11.

In fig. 6, in another implementation, a schematic representation in section is shown, in which the lens 11 is held horizontally with its center-plane I in the cassette 8 by the position holding device. Furthermore, the movement channel 121 is disposed inclined thereto with its longitudinal axis A, namely in an angle α. The movement channel 121 directly opens to the recess 26 of the haptic part 18 such that the plunger 4 in turn can directly engage with the optic part 16 and/or the first section 27 in the region of the edge 33 for advancing the lens 11, without previously having contacted the lateral edge 31 and without having deformed the second section 28 in particular in x-direction.

## Claims

1. Cassette for containing an intraocular lens (11), wherein said cassette (8) is configured to be loaded into a lens injector device (1), the cassette comprising an entry region (12) for a plunger (4) of the lens injector device (1) and an exit region (15) for the intraocular lens (11), **characterized in that** said cassette (8) includes a movement channel (121) for the plunger (4), wherein a longitudinal axis (A) of the movement channel (121) and a center-plane (I) of the intraocular lens (11) are oriented inclined to each other in the state loaded in the cassette (8) in a rest-position of the intraocular lens (11).

2. Cassette according to claim 1, **characterized in that** the longitudinal axis (A) extends in a horizontal plane (II) of the cassette (8) and the intraocular lens (11) is positioned inclined with respect to the horizontal plane (II) in a rest-position in the cassette (8).

3. Cassette according to claim 1 or 2, **characterized in that** a position holding device (42) for the intraocular lens (11) is formed, by which an inclined rest-position of the intraocular lens (11) in the cassette (8) can be held, especially **in that** the cassette (8) has a cover part (9) and a base part (10) movable relatively thereto, and the position holding device (42) is formed by elements of the cover part (9) and/or the base part (10), wherein, for this, the cover part (9) and/or the base part (10) have a shaping at least in certain regions on their surfaces facing the lens (11), which result in an inclined rest-position of the lens (11) in the closed state of the cassette (8) with the lens received.

4. Cassette according to claim 3, **characterized in that** the position holding device (42) is formed such that a tolerance of movement for the intraocular lens (11) in the rest-position is formed.

5. Cassette according to any one of claims 3 or 4, **characterized in that** the position holding device (42) is formed such that the lens (11) is disposed inclined to the bottom in the rest-position based on the end of the lens (11) facing the entry region (12) of the cassette (8).

6. Cassette according to any one of claims 3 to 5, **characterized in that** the rest-position of the intraocular lens (11) in the cassette (8) is formed by the position holding device (42) such that the center-plane (I) and the longitudinal axis (A), in particular a horizontal plane (II) having the longitudinal axis (A), intersect in the region of an outer edge (21) of an optic part (16) of the intraocular lens (11), which has a recess (19, 26) in a haptic part (17, 18), or a first section (27) of a haptic part (18) of the intraocular lens (11) directly abutting on an edge (21) of the optic part (16).

7. Cassette according to claim 5 or 6, **characterized in that** the rest-position is formed such that for advancing the lens (11) out of the cassette (8), the plunger (4) is disposed in non-contacting manner with the outer edge (31) of the second section (28) of the haptic part (18).

8. Cassette according to claim 6 or 7, **characterized in that** the lens (11) is disposed in the cassette (8) in its rest-position such that a second section (28) of the haptic part (18) is disposed above the movement path of the plunger (4), in particular a bottom (30) of the second section (28) rests on the plunger (4) after releasing the rest-position of the lens (11).

9. Cassette according to claim 7 or 8, **characterized in that** the position holding device (42) and the movement channel (121) are formed such that in a first phase of advancing the lens (11) out of the cassette (8), the plunger (4) is contacted with a bottom (30) of the second section (28) of the haptic part (18) for lifting the lens (11), and in a second phase of advancing-out, the plunger (4) is contacted with the first section (27) of the haptic part (18) and/or the optic part (16).

10. Cassette according to any one of claims 3 to 9, **characterized in that** the position holding device (42) has at least one form element (34), which has a lay-on surface (36), on which the intraocular lens (11) can be laid with a haptic part (18) in the rest-position, especially that the lay-on surface (36) of the form element (34) is respectively limited at the front and at the back by an elevation (37, 38), and/or that the form element (34) is disposed on a cover part (9) or a base part (10) of the cassette (8).

11. Cassette according to anyone of the preceding claims, **characterized in that** the cassette (8), in particular the position holding device (42), is formed for receiving an intraocular lens (11), which has two haptic parts (17, 18), which are formed on opposing positions of an optic part (16), wherein the rest-position of the lens (11) in the cassette (8) is formed such that the longitudinal axis (A) of the movement channel (121) extends through the two haptic parts (17, 18) or is an axis of symmetry of the lens (11) through the haptic parts (17, 18) in a plane with the longitudinal axis (A) in case of a position of the lens (11) inclined with respect to the horizontal plane (II).

12. Lens injector device (33) comprising a cassette (8) according to any one of claims 1 to 14, in which an intraocular lens (11) is disposed, and having a movable plunger (4) formed for advancing the intraocular lens (11) out of the cassette (8) and for advancing it into an insertion channel (7) of the lens injector device (33).

13. Method for advancing an intraocular lens (11) out of a cassette (8) according to any one of claims 1 to 14, in which a plunger (4) of a lens injector device (1) is advanced through an entry region (12) of the cassette (8) to the lens (11), and upon advancing the lens (11) out of the cassette (8) through an exit region (15) of the cassette (8), the plunger (4) is moved in non-contacting manner with an edge (31) of a second section (28) of the haptic part (18) of the lens (11).

14. Method according to claim 13, **characterized in that** upon advancing the lens (11) out of the cassette (8), only the first section (20, 27), in particular the edge (32, 33) thereof, of the haptic part (17, 18) of the lens (11) and/or the optic part (16) of the lens (11) is contacted by the plunger (4).

15. Method according to claim 13 or 14, **characterized in that** in a first phase of advancing-out, the plunger (4) is contacted with the bottom (30) of the second section (28) of the haptic part (18) and the lens (11) is lifted, and in a second phase of advancing-out, the plunger (4) is contacted with the first section (27) of the haptic part (18) and/or the optic part (16) and the lens (11) is advanced out, especially that after lifting from the rest-position via an elevation (38) of the form element (34), the lens (11) is advanced forward with the plunger (4) over this elevation (38) and is lowered again and is laid on the plunger (4) with the bottom (30) of the second section (28) of the haptic part (18) and is further advanced out.
